# EUROPEAN PATENT APPLICATION

(11) **EP 2 090 325 A2**
(43) Date of publication of application: **19.08.2009**
(21) Application number: 07852039.2
(22) Date of filing: 30.10.2007
(51) Int. Cl.: A61L 9/22

(54) **METHOD FOR DISINFECTING AIR USING NEGATIVE OXYGEN IONS AND A DEVICE FOR CARRYING OUT SAID METHOD**

(30) Priority: 07.11.2006 RU 2006139085
(71) Applicant: Churkin, Andrey Andreevich, Novosibirsk 630-054 (RU); Choporov, Vasily Egorovich, Novosibirsk 630-090 (RU); Khoroshilov, Vladimir Nikolaevich, Novosibirsk 630-089 (RU); Ladychenko, Elina Leonidovna, Moscow 119-334 (RU)
(72) Inventor: Churkin, Andrey Andreevich, Novosibirsk 630-054 (RU); Choporov, Vasily Egorovich, Novosibirsk 630-090 (RU); Khoroshilov, Vladimir Nikolaevich, Novosibirsk 630-089 (RU); Ladychenko, Elina Leonidovna, Moscow 119-334 (RU)
(74) Representative: Arth, Hans-Lothar
(86) International application number: PCT/RU2007/000600
(87) International publication number: WO 2008/057010

(57) **Abstract**

A method and device are related to air treatment and purification, and more particularly to air and aerosol disinfection by inactivating and destroying bacteria, fungi and viruses including of avian flu H5N1 virus during one passage of the air and aerosol flow through inlet system of ventilation before blowing disinfected air and aerosol into premises of residential, commercial and industrial buildings.

A method for disinfecting air using negative ions of oxygen comprising the steps of (a) blowing the air through the ventilation system, (b) exposing the air to the flow of alpha particles, (c) reducing positive ions contained in the air to neutral molecules at the surface of an electrode having negative electric potential, (d) simultaneously decreasing the energy of free electrons contained in the air to the range of 0.4 to 2.0 eV through effect of electric field that is parallel to the air flow, (e) providing high concentration of negative ions of oxygen for at least 4 to 36 seconds during which time bacteria, fungi and viruses including avian flu virus contained in the air and aerosol are inactivated and destroyed, (f) reducing concentration of negative ions of oxygen to the range of 600 to 50000 sm⁻³ with a unipolarity coefficient, that is number of positive ions of oxygen divided by number of negative ions of oxygen, approximating 0 at the surface of the electrodes having positive electric charge before blowing disinfected air and aerosol into the premises.

A device for disinfecting air using negative ions of oxygen is presented that consists of serially connected air inlet duct of ventilation system, ionization chamber, disinfection chamber, air outlet, whereas in one of the embodiments the disinfection chamber is divided into discharge duct connected to the atmosphere and air distribution duct connected to air outlets through which disinfected air is blown into the premises.

Ionization chamber contains at least one source of alpha particles mounted on at least one plate. At least two electrodes are mounted across air flow after the plate at the back of ionization chamber. Ionization chamber and plates are made of a non-conducting material. Each electrode is made of a conducting mesh connected to negative terminal of direct voltage source, the positive terminal of which is earthed. Each plate has a depression in which sources of alpha particles are fixed provided depth of the depression is at least 2 mm greater than thickness of a source of alpha particles. The distance between the last source of alpha particles along the ionization chamber and the electrode nearest to it does not exceed 20 mm. Voltage on electrodes is maintained within the range of 0.2 to 10.0 kV depending on the size of cells of the mesh.

Disinfection chamber is a part of the ventilation duct where air has a high concentration of negative ions of oxygen. The length of the disinfection chamber is a distance between the last electrode mounted at the back of ionization chamber and the nearest electrode mounted in outlet duct used to distribute disinfected air into premises. This length *L* is no less than *L*≥*TV,* where *V* - mean linear velocity of air flow in the disinfection chamber and time *T* is *5.0*≤*T*≤*36.0*s.

In one of the embodiments of the device the disinfection chamber, being part of the ventilation air duct, is divided into two branches: distribution and discharge ducts. At least one electrode is mounted in the outlet duct. The electrode is made of a conducting mesh connected to a positive terminal of direct voltage source, the negative terminal of which is earthed. The voltage on the electrode is 1.0 to 10.0 kV.

The discharge duct has an electrode installed at its entry. The electrode is made of a mesh mounted across the air flow. It is connected to the negative terminal of direct voltage source, whereas positive terminal of it is earthed. The voltage on the electrode is 1-10 kV depending on dimensions of cells of the mesh.

## Description

### BACKGROUND OF THE INVENTION

### 1. TECHNICAL FIELD

The present invention relates to a method and device for disinfecting air and aerosol media using negative ions of oxygen to inactivate and destroy highly pathogenic microorganisms and viruses including virus of avian influenza H5N1. Invention can be used in forced ventilation systems of residential, commercial and industrial buildings.

The terms and phrases used below have the following meaning:
*Disinfection* means treatment of media or premises in order to destroy or inactivate pathogenic microorganisms, fungi and viruses.
*Inactivation* means partial or complete loss of activity by a biologically active substance or an agent, i.e. depriving them of an ability to reproduce.
*Disinfected premise* means one or several premises in which people, poultry, cattle or animals can stay during disinfection.
*Disinfected air* or just "air" mean gaseous medium subjected to disinfection provided air contains dust or aerosol with highly pathogenic viruses and microorganisms.
*Unipolarity coefficient* is a relation of concentration of aero-ions with positive polarity to a concentration of aero-ions with negative polarity.
*Time of disinfection* is a lapse of time during which the air and aerosols are disinfected by negative ions of oxygen.

### 2. DESCRIPTION OF THE PRIOR ART

Various methods of air disinfection with the use of ions have been proposed to destroy or inactivate pathogens in the air.

A number of methods use ionization of air by bombarding the air with a beam of electrons originating from gas discharge at a high voltage of electric field. For example, A.L.Chizhevsky developed a luster of glowing discharge known from his book: A.L. Chizhevsky's book "A Guide on Application of Ionized Air", Moscow, Gosplanizdat, 1959 [1]. However there is a number of drawbacks of the glowing discharge method. For example, the method has a low disinfection rate because ions fall out quickly on negative electrode and surfaces of objects in a premise and do not contact with the air. The method requires high voltage of about 100 kV and produces high concentration of ozone and nitrogen oxides.

There are a number of patents concerning application of discharge methods for air ionization. For example, the Russian patent RU2021822 [2] concerns a method and device for air ionization comprising igniting gas discharge in the air (corona discharge, glowing discharge or electric arc). The disadvantage of this method is that high concentrations of ozone and nitrogen oxides are generated which exceed maximum permissible level. Further problems of these methods consist in high rate of electricity consumption whereas most of the energy is spent on heating the air and burning oxygen.

To solve the discharge method problems a number of inventions have been developed which are based on application of ionization with the use of radio-active isotope with alpha-decay. These methods do not produce oxygen or nitrogen oxides in the air. For example, the Patent JP 3221076 [3] discloses storing an ion generating material containing therein a radio-active isotope with alpha- decays, in a container, and introducing ions generated from the material and discharged from the container, into an ionization room or into an ionization utilizing facility by means of a blower. However these methods have a number of shortages main of which is a low and indefinite concentration of negative ions of oxygen. This is because free electrons, which are released when alpha particles collide with air molecules, recombine quickly with positive ions generated by alpha particles knocking out electrons from them. So the free electrons do not have sufficient time and energy to combine with the molecules of oxygen to form negative ions of oxygen. Thus disinfecting effect is very low.

There are a number of patents which disclose ionization methods that control energy of electrons. For instance, the Russian Patent RU2061501 [4] describes gas ionizer consisting of a generating electrode with metal needles connected with a high voltage source, an extract electrode and at least one accelerating electrode connected with the generating electrode via voltage divider. The generating electrode form free electrons, then negative ions are collected from the area of air ionization close to the generating electrode, after that these ions are accelerated and blown into the volume of air that should be treated. However such methods have a number of drawbacks. For example, the methods require high voltages of at least 25 kV. These methods have a low disinfection rate because ions flow along electric field lines and do not cover entire disinfected volume of air. The result is that the air is actually treated by ozone and nitrogen oxides instead of by negative ions of oxygen. Further problem of these methods is high concentration of ozone and nitrogen oxides which is usually higher than maximum permissible concentration. This air can be toxic to people, poultry and animals staying in premises and therefore such methods are not allowed for application due to requirements of hygienic and healthcare standards. The air with such a high concentration of ozone and nitrogen oxides is prohibited to be blown into residential or industrial premises.

### SUMMARY OF THE INVENTION

The purpose of the present invention is to overcome the above-mentioned drawbacks and more particularly to increase air disinfection rate and improve quality of air disinfection. The solution of these problems is directed at creating the method for achieving the following technical effects: (a) inactivation of 100% of viruses and pathogenic microorganisms contained in the air and aerosol before the air is blown into premises through ventilation system during one passage of air through the air feeding ventilation system and (b) partial inactivation of viruses and pathogenic microorganisms which are already in the premises. Another technical problem solved by the present invention is prevention of abrasive wear of a source of alpha-particles by abrasive particles contained in the air.

These technical effects are obtained in the embodiment of the method that includes (a) treatment of air by a stream of alpha particles, (b) reduction of positive ions contained in the air after treatment by alpha particles to neutral molecules, (c) simultaneous reduction of energy of free electrons in the air to the level of 0,4 to 2,0 eV at which an electron attachment and trapping takes place between free electrons and molecules and atoms of oxygen within the range of energies of oxygen's affinity to electron, (d) then the air including aerosol with viruses and pathogenic microorganisms is treated by the air containing negative ions of oxygen for 4 to 36 seconds, (e) immediately before blowing the air into premises the concentration of negative ions of oxygen is reduced.

Also, positive ions of air are reduced to neutral molecules at the surface of an electrode having negative electric charge, energy of free electrons contained in the air is reduced by electric field that is parallel to the air flow, providing forming of negative ions of oxygen whereas concentration of negative ions of oxygen is reduced before the air is blown into premises by reducing the negative ions of oxygen to neutral molecules of oxygen at the surface of an electrode having positive electric charge.

Also, by reducing a part of negative ions of oxygen to neutral molecules the concentration of negative ions of oxygen in the air is provided within the range of 600 to 50,000 cm⁻³ with an unipolarity coefficient approximating 0 directly before blowing the disinfected air into premises.

Inactivation of 100% of viruses and pathogenic microorganisms is provided by creating a high concentration of negative ions of oxygen in the disinfected air provided the concentration reaches the level of at least 20·10⁶ cm⁻³. This makes it possible to inactivate up to 100% of viruses and pathogenic microorganisms them within the shortest time possible due to oxidation effect. The presented embodiment of the invention allows to inactivate about 100% of viruses and pathogenic microorganisms within the period of time of 4.0 to 36.0 seconds.

Above-mentioned concentration of negative ions of oxygen in the air is provided the following way. First, disinfected air is processed by a stream of alpha particles, causing the impact ionization of air molecules and resulting in forming positive ions and free electrons. Then the free electrons are trapped by neutral molecules and atoms of oxygen, these electrons attach to these molecules and atoms resulting in forming negative ions of oxygen in the air, provided that the energy of free electrons lies within the range of energy of oxygen's affinity to electron that is 0.4 .. 2.0 eV.

To maintain a high concentration of free electrons, recombination of free electrons with positive ions must be prevented. Positive ions are reduced to neutral molecules on one or more electrodes which are made in a form of a conducting mesh, through which the air flows. To ensure that negative ions of oxygen are formed through the process in which molecules of oxygen trap and attach free electrons, a negative electric charge is supplied to the conducting mesh from a direct voltage source. A potential barrier created by negatively charged mesh reduces kinetic energy of electrons to the energy of electron's affinity to a molecule and an atom of oxygen, which allows them to interact effectively with oxygen of the air. This results in forming negative ions of oxygen.

In order to prevent electric short-circuit the parts of the device should be manufactured of non-conducting material.

Negative ions of oxygen provide disinfecting effect during the period of 4.0 - 36.0 seconds. This period of time is determined through a conversion of linear velocity of the air flow in the ionization chamber where sources of alpha particles are mounted into the volume velocity (air rate) of air flow in an air disinfection chamber.

Before blowing the air into premises, where animals, poultry and people can stay, it is essential to reduce the concentration of negative ions of oxygen to a level that meets healthcare standards, notably 600 to 50,000 cm⁻³ with a unipolarity coefficient approximating 0. To accomplish this, a part of negative ions of oxygen are reduced to neutral molecules on one or more electrodes made of conducting mesh, through which disinfected air flows and at which positive electric potential is supplied by direct voltage source.

The present method is embodied in a device comprising consecutively interconnected air inlet duct, an ionization chamber, an air disinfection chamber and an air outlet for distribution of disinfected air into premises whereas at least one source of alpha particles is mounted in the ionization chamber by mounting the source of alpha particles on at least one plate. Two electrodes are mounted at the entrance of the disinfection chamber behind the plate along the air flow, whereas the electrodes are made of conducting mesh mounted across the air flow and connected to a negative terminal of a direct voltage source, the positive terminal of which is earthed.

Also, at least one electrode made of conducting mesh is mounted in the air outlet used for distribution of the air into premises, whereas the said electrode is connected to a positive terminal of direct voltage source, the negative terminal of which is earthed.

Also, the walls of the ionization chamber, air disinfection chamber and the plates are made of non-conducting material.

Also, depressions are made in the plate in which the sources of alpha particles are mounted, whereas the depth of a depression is at least 2 mm greater than the thickness of the source of alpha particles.

Also, the distance between the alpha particles source and the nearest electrode is less than 20 mm.

Also, the voltage at the electrodes is within 0.2 - 10 kV range whereas exact voltage is selected depending on the size of the cells of the mesh electrode.

Also, the length *L* of the air duct constituting the air disinfection chamber where negative ions of oxygen are used whereas the air disinfection chamber ends at the nearest electrode mounted in the air outlet for distribution of disinfected air into premises is greater than *L* ≥ *TV*, where *V* is an average linear velocity of air flow in the air outlet - disinfection chamber, and the time *T* is in the range of *4.0* ≤ *T* ≤ *36.0* seconds.

Also, at least two mesh electrodes are mounted across air flow in the space between sources of alpha particles and the air outlet duct, i.e. in the air disinfection chamber, whereas the said electrodes are connected to the negative terminal of direct voltage source, the positive terminal of which is earthed. Due to that the positive ions of air are reduced to neutral molecules at the first said electrode. The energy of free electrons contained in the air is reduced to a required level that depends on the negative potential maintained on the mesh electrode and on the distance between the electrode and the source of alpha particles nearest to each other.

Mounting the mesh electrode and the nearest source of alpha particles at a distance of no more than 20 mm provides the distribution of electrons by velocities with an average energy in the range of 0.4 to 2.0 eV. This range of energy provides trapping of free electrons by molecules and atoms of oxygen and attachment of trapped electrons to atoms and molecules of oxygen due to the energy of electrons being within the electron's energy affinity to oxygen. This forms negative ions of oxygen.

In order to treat the air by the largest possible number of alpha particles at a given number of alpha particles sources, the alpha particles sources are mounted on the plates located parallel to the air flow.

Free path of alpha particles in the air depends on their energy defined by an isotope used in the alpha particles source. Free path of alpha particles in the air is several centimeters. This value can be determined from standard tables. For example, an isotope P₂₃₉ has a maximum energy of emitted alpha particles at the level of 5.1 MeV, whereas an average free path of alpha particles in the air is 50 mm.

A distance between the plates with sources of alpha-particles, as well as a distance between the extreme plates with sources of alpha-particles and side walls of the ionization chamber should not exceed a free path of alpha particles. Otherwise, entire volume of air will not be treated by alpha particles. In one of the embodiments when sources of alpha particles are mounted on both sides of the plates, the entire air will be treated by alpha particles, provided the gap between the plates is no more than double free path of alpha particles in the air.

Mounting sources of alpha particles in the depressions of the plates allows to avoid abrasive wear of alpha particles sources and tear of isotope particles by abrasive particles contained in the air flow. This also allows to select the optimal energy of alpha particles.

At least one mesh electrode mounted across the air flow in the air outlet used for distribution of air in premises is connected to a positive terminal of direct voltage source, the negative terminal of which is earthed. It provides a reduction in the concentration of negative ions of oxygen contained in the disinfected air by reducing negative ions of oxygen to neutral molecules at above-mentioned electrode. The concentration of negative ions of oxygen in the air blown into the premises is determined by the level of positive voltage at the said electrode, the size of cells of the mesh and by air velocity.

The length *L* of air disinfection chamber is determined from condition of ensuring adequate time in the range of 4.0 ≤ T ≤ 36.0 seconds for complete disinfection of air and inactivation of approximately 100% of viruses and pathogens. The technical effect achieved within the specified time, is shown in the table below.

The above-mentioned range of output voltage of direct voltage sources are determined by a calculation in each particular case and confirmed by experiments. These data allow to select the best mode of operation of the present device for particular dimensions of the device and airflow velocity. At the same time such an embodiment provides easy manufacture and maintenance as well as low cost of power sources. Typically, it is appropriate to use parallel connection of several electrodes to one power source or to combine several power sources in one device to supply power to several electrodes.

Also, the present invention provides another embodiment of the device for disinfecting air using negative ions of oxygen, comprising disinfection chamber that is divided into two branches, one of which is air discharge duct connected to the atmosphere, whereas the other is connected to the ductwork used to distribute disinfected air into premises, whereas a mesh electrode is mounted across the air flow at the entrance to the air discharge duct connected to the atmosphere. This electrode is connected to the negative terminal of direct voltage source, the positive terminal of which is earthed.

Also, the voltage on the mesh electrode mounted at the entrance to the air discharge duct connected to the atmosphere is at least 1 to 10 kV and is determined depending on the size of cells of the mesh electrode.

The present embodiment of the device allows to increase concentration of negative ions of oxygen in the disinfection chamber due to separation of a part of the airflow and blowing it into the atmosphere through the air discharge duct, whereas negative ions of oxygen remain in disinfection chamber and the outlet air duct used for the distribution of air into premises due to repulsion of negative ions of oxygen from negatively charged mesh electrode mounted across the air flow at the entrance to the outlet air duct, whereas the negative charge of the said mesh electrode is formed by connecting it to the negative terminal of direct voltage source, the positive terminal of which is earthed.

### BRIEF DESCRIPTION OF THE DRAWINGS

FIG.1 shows the construction of the device for disinfecting air using negative ions of oxygen according to the present invention installed in the ventilation system of a building.
FIG.2 is an explanatory drawing showing schematically an experimental device used in experimental and industrial trials of the method and the device.

### DETAILED DESCRIPTION OF THE PREFERRED EMBODIMENTS

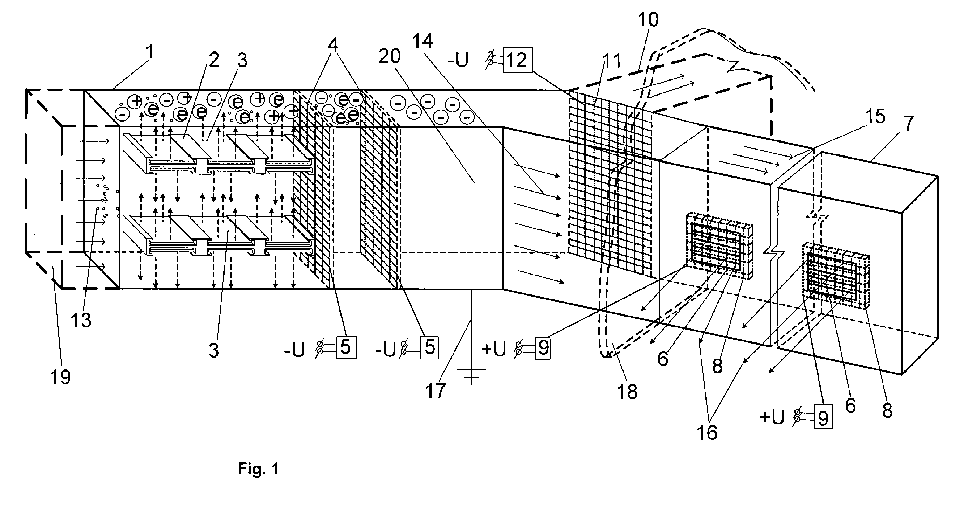

FIG. 1 shows the construction of the device for disinfecting air using negative ions of oxygen installed in an air supply system such as a ventilation system of a building or the like.

As shown, an ionization chamber 1 can be made as a part of an air inlet duct 19 in which dielectric plates 2 are mounted in parallel along the air inlet duct. Sources 3 of alpha particles are mounted in the depressions in the surfaces of dielectric plates 2. Sources 3 of alpha particles are made in the form of substrates onto which sealed sources of alpha - decay are embedded.

A distance between the dielectric plates 2 across the air flow in the chamber 1 does not exceed 100 mm. This distance corresponds to double length of an average free path of alpha particles having energy of 5.1 MeV in the air, whereas a distance between the extreme dielectric plates and the walls of the ionization chamber does not exceed 50 mm.

Also, two electrodes 4 are mounted in the ionization chamber 1. The electrodes 4 are made as a conducting mesh mounted in the insulated frame and connected to a negative terminal of direct voltage source 5, the positive terminal of which is earthed.

Also, electrodes 8 are mounted in the air outlets 6 of an air duct 7 used to blow disinfected air into premises whereas electrodes 8 are made similar to electrodes 4, but the mesh of electrodes 8 is connected to a positive terminal of direct voltage source 9, a negative terminal of which is earthed.

FIG.1 shows an embodiment of the device with an additional branch 10 of the air duct whereas the branch 10 is used to discharge a part of the air flow into atmosphere. An electrode 11 is made in a manner similar to electrodes 4 and electrodes 8 and is mounted at the entrance to the branch 10. A negative electric potential of up to 10 kV is maintained at the electrode 11 by the direct voltage source 12.

The length *L* of an air disinfection chamber 20 that ends at the nearest electrode mounted in the air outlet made for distribution of disinfected air into premises is greater than *L* ≥ *TV,* where *V* is the average linear flow rate of air in the disinfection chamber, and time *T* is in the range of 4.0 ≤ *T* ≤ 36.0 seconds.

For instance, if the cross section of the disinfection chamber is 0.7 x 0.7 m, an air flow rate is 32 m³/hr, the average linear flow rate of air in the air disinfection chamber will be 0,018 m/s, then the length of the disinfection chamber must be no less than *L* > *0.65* m for the air to be disinfected during 36 seconds.

The embodiment of the device shown in FIG. operates the following way. The air 13 to be disinfected flows into the device through the air inlet duct 19 from which it gets into the ionization chamber 1, where the air goes through a stream of alpha particles emitted by sources 3.

As a result of impacts of the air molecules with alpha particles, the air molecules are reduced by electrons and the air is transformed into positive ions and a cloud of free electrons. Then the air passes successively through the electrodes 4, at which positive ions are reduced to neutral molecules whereas free electrons slow down to an energy of 0.4 - 2.0 eV. In this range of energies the free electrons are trapped by molecules and atoms of oxygen and attach to them forming negative ions of oxygen. This process yields the air with a high concentration of negative ions of oxygen with high disinfecting power. Air 14 enriched with negative ions of oxygen is processed during 4.0 ≤ *T* ≤ 36.0 seconds within the disinfection chamber 20 by negative ions of oxygen at the length *L.* In the embodiment of the device where additional branch 10 of the air ductwork is applied, a part of the air flow is discharged into the atmosphere whereas negative ions of oxygen remain in the device due to being repulsed from negatively charged electrode 11. As a result higher concentration of negative ions of oxygen is achieved.

If disinfected air 15, while passing the outlet air duct 7 used for distribution of disinfected air into the premises, has a high concentration of negative ions of oxygen according to healthcare standards, a part of the negative ions of oxygen is reduced to neutral molecules at the electrodes 8. Disinfected air 16 with a concentration of negative ions of oxygen that meets the healthcare standards is fed into the premises.

Fine-tuning of the device made in order to provide the most effective disinfection of air is achieved by selecting the following parameters: output voltage supplied to mesh electrodes, the size of the cells of mesh electrodes and by the depth of depression into which substrates with alpha particles are installed.

If the walls of the air duct are made of conducting materials, then in order to maintain the required concentration of negative ions of oxygen along the entire ductwork that distributes disinfected air into premises, as well as in order to provide electrical safety of the device, the air duct is earthed by electrical grounding 17.

### EXPERIMENTAL AND INDUSTRIAL RESULTS

Industrial application of the present method and device for disinfecting air using of negative ions of oxygen was tested by the Federal State Scientific Institute - the State Research Centre "Vector" of the Federal Service for Supervision of Consumer Rights Protection and Human Welfare. The technical effect was confirmed by testing three experimental and industrial pilot devices.

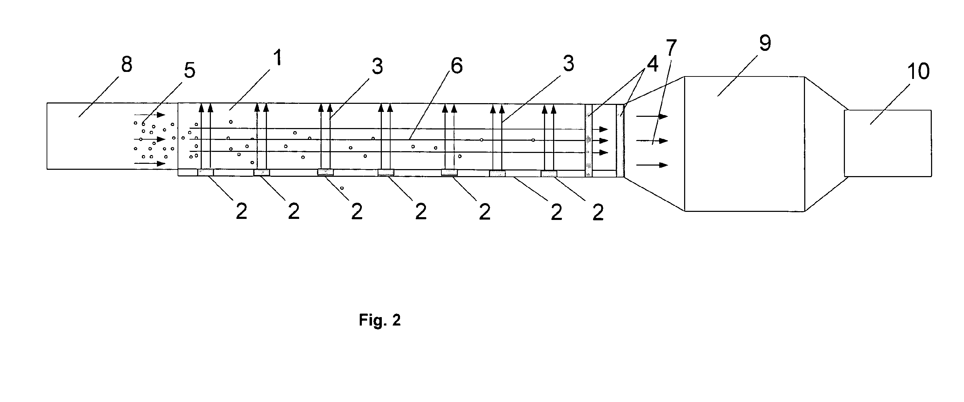

Experimental and industrial device as shown in FIG.2 comprised an air feeding duct 8, through which the air 5 to be disinfected entered the ionization chamber 1. The dielectric plate was installed in the ionization chamber 1. In different industrial prototypes there were 1 to 10 sources of alpha particles 2 mounted linearly on the dielectric plate whereas each of the sources of alpha particles comprised a substrate containing Plutonium 239 manufactured according to GOST R51873-2002 (Sealed Sources of Ionizing Radiation. General Specifications). Maximum energy of alpha particles 3 emitted from the sources applied in tests was 5.1 MeV. It corresponded to the average free path of alpha particles in the air of 50 mm. The flow of alpha particles 3 was perpendicular to the air flow 6.

Electrodes 4 were mounted at a distance of 20 mm from the source of alpha particles that is the last source along the air flow. The electrodes 4 were made of a metal mesh mounted in the non-conducting frame and connected to the negative terminal of direct voltage source, the positive terminal of which was earthed. The walls of the air duct were also earthed. The total length of the device was 2605 mm, whereas the length of the ionization chamber 1 with the sources of alpha particles and the electrodes mounted in it was 1505 mm, the cross section of disinfection chamber was 60 mm x 50 mm, the length of disinfection chamber 9 was 1100 mm, area of the cross section was 700 mm x 700 mm. Disinfection chamber 10 was connected to distribution ductwork 11.

This embodiment of the method was tested in a series of experiments involving 3 tests with an airflow rate of 32.5 m³/h. The tests used an aerosol containing highly pathogenic avian flu virus (strain A/Chicken/Suzdalka/Nov-11/2005, extracted by the staff of SRC VB "Vector" on 27.07.05 during the epizootic avian flu in chickens in the Novosibirsk region, Russia) with the original activity of 8 , 0 - 6,0 1g TTsD50/ml. Virus containing suspension was used as dispersible liquid.

Biological activity of the virus in samples was determined by cytopathogenic effect of the virus on sensitive cell culture MDCK. Mass concentration and parameters of FDS of aerosol were calculated basing on the results of fluorescent analysis. Final results are shown in the table below.

The tests have shown that the presented method and device provided high efficiency of inactivation of the avian flu virus (strain A/Chicken/Suzdalka/Nov-11/2005) during one passage of contaminated air flow through the device with the result of 99,983 ± 0005% at the airflow rate of 32,5 m³/ h. At the same time, the impact of the flow of alpha - parts on air containing the avian flu virus ranged from 5 to 8 seconds.

These government tests have shown that a sequential linear placement of sources of alpha particles in systems with a high linear velocity of airflow sources did not have strong impact on efficiency of the method and device (see table data for 1, 3, 7 and 10 sources of alpha particles) whereas conversion of linear velocity of air in the chamber in which sources of alpha particles are mounted into the volume velocity of air flow in air duct that represents the disinfection chamber is the determining factor (see Table).

**Table. The results of tests of the device for the presented method of disinfecting the air with the use of negative ions of oxygen from the highly pathogenic avian flu virus of type A (H5N1) by the Federal State Scientific Institute - the State Research Centre "Vector" of the Federal Service for Supervision of Consumer Rights Protection and Human Welfare**

| Point of air sampling | Efficiency of aerosol filtration by weight, % | Air disinfection efficiency, % | Efficiency of avian flu inactivation, % |
|---|---|---|---|
| At the entry of the device | 0 | 0 | 0 |

| Tests, stage I | | | |
|---|---|---|---|
| After 3^{rd} source of alpha-particles of the device | 9,5 ± 8,0 | 7882 ± 25,15 | 76,60 ± 27,93 |
| After 7^{th} source of alpha-particles of the device | 26,3 ± 7,6 | 94,05 ± 4,06 | 91,95 ±5,51 |
| At the outlet (7 sources of alpha-particles) of the device | 24,7 ± 6,0 | 99,76 ± 0,14 | 99,58 ± 0,22 |

| Tests, stage II | | | |
|---|---|---|---|
| At the outlet (10 sources of alpha-particles) of the device | 31,6 ± 8,0 | 99,988 ± 0,003 | 99,983 ± 0,005 |

| Tests, stage III | | | |
|---|---|---|---|
| At the outlet (1 source of alpha-particles) of the device | | 99,972 ± 0,007 | 99,957 ± 0,005 |

| Control tests without sources of alpha-particles | | | |
|---|---|---|---|
| At the outlet of the device | | 84,61 ± 6,47 | 77,94 ± 9,13 |

### REFERENCES

1. Chizhevsky A.L. A Guide on Application of Ionized Air, Moscow, Gosplanizdat, 1959.
2. Russian Patent RU2021822, publication date 30.10.1994.
3. Japan Patent JP3221076, publication date 30.09.1991.
4. Russian Patent RU2061501, publication date 10.06.1996.

## Claims

1. A method for disinfecting air using negative ions of oxygen by treating air and aerosol media by a flow of alpha particles, comprising the steps of:
- Reducing the positive ions contained in the air to neutral molecules after the air has been treated by alpha particles;
- Simultaneously reducing an energy of free electrons contained in the air so that the energy of the electrons fits in the range of 0.4 to 2.0 eV providing forming of negative ions of oxygen;
- Treating the air by negative ions of oxygen during 4.0 to 36.0 seconds;
- Reducing the concentration of negative ions of oxygen in the air before blowing the air into premises.

2. The method according to Claim 1, wherein the positive ions contained in the air are reduced to neutral molecules on the surface of an electrode having a negative charge, the energy of free electrons contained in the air is reduced by the action of the electric field that is parallel to the air flow providing forming of negative ions of oxygen, and the concentration of negative ions of oxygen is reduced by reducing negative ions of oxygen to neutral molecules of oxygen on the surface of an electrode having a positive charge before blowing the air into premises.

3. The method according to Claim 1, wherein by reducing a part of negative ions of oxygen to neutral molecules the concentration of negative ions of oxygen in the air is reduced to a range of 600 to 50000 cm⁻³ with a unipolarity coefficient close to 0 before the disinfected air is blown into premises.

4. A device for disinfecting air using negative ions of oxygen, comprising
- a series connection of an air inlet, ionization chamber, an air disinfection chamber and an air outlet for distribution of disinfected air into the premises,
- at least one source of alpha particles mounted in the ionization chamber by fixing it on at least one plate,
- at least two electrodes made of conducting mesh mounted across the air flow after the plate with the source of alpha particles along the flow of air in the disinfection chamber and connected to a negative terminal of direct voltage source, the positive terminal of which is earthed,
- at least one electrode made of conducting mesh mounted in the air outlet for distribution of disinfected air into premises, whereas the electrode being connected to a positive terminal of direct voltage source, a negative terminal of which is earthed,
- whereas the walls of the disinfection chamber and a plate are made of non-conducting material, a plate has a depression in which the source of alpha particles is fixed provided the depth of the depression is at least 2 mm greater than the thickness of the source of alpha particles, and the distance between the electrode nearest to a source of alpha particles and this source of alpha particles does not exceed 20 mm, wherein the voltage at the electrodes is within the range of 0.2 - 10 kV and is selected depending on the size of the cells of the mesh electrode.

5. The device for disinfecting air using negative ions of oxygen according to Claim 4, whereas the length *L* of air duct that is the air disinfection chamber that ends at the nearest positively charged electrode, which is located in the air outlet for distribution of disinfected air to the premises is no less than *L* ≥ *TV*, where *V* is the average linear flow rate of air in the disinfection chamber, and time *T* is 4.0 ≤ *T* ≤ 36 seconds.

6. A device for disinfecting air using negative ions of oxygen comprising:
- a series connection of an air inlet, ionization chamber, an air duct being the air disinfection chamber and an air outlet for distribution of disinfected air into the premises,
- at least one source of alpha particles mounted in the ionization chamber by fixing it on at least one plate,
- at least two electrodes made of conducting mesh mounted across the air flow after the plate containing a source of alpha particles along the flow of air in the disinfection chamber and connected to a negative terminal of direct voltage source, the positive terminal of which is earthed,
- at least one electrode made of conducting mesh mounted in the air outlet for distribution of disinfected air into premises, whereas the electrode being connected to a positive terminal of direct voltage source, a negative terminal of which is earthed,
- whereas the air outlet is divided into two branches one of which has an outlet to the atmosphere and another is connected with an air outlet used for distribution of disinfected air into premises, wherein an electrode is mounted at the entry to the branch connected with the atmosphere, whereas the electrode is made of conducting mesh mounted across the air flow and connected to a negative terminal of direct voltage source, the positive terminal of which is earthed.

7. The device for disinfecting air using negative ions of oxygen according to Claim 6, whereas the voltage at the mesh electrode in the branch connected to the atmosphere is 1 to 10 kV and selected depending on the size of the cells of the mesh electrode.
